# EUROPEAN PATENT APPLICATION

(11) **EP 4 684 753 A1**
(43) Date of publication of application: **28.01.2026**
(21) Application number: 24765008.8
(22) Date of filing: 12.06.2024
(51) Int. Cl.: A61B 90/00, A61B 17/072, A61B 18/14

(54) **INTERVENTIONAL INSTRUMENT, INTERVENTIONAL INSTRUMENT CONTROL METHOD, COMPUTER-READABLE STORAGE MEDIUM, AND CONTROLLER**

(30) Priority: 07.06.2024 CN 202410735280
(71) Applicant: Ningbo Verykind Medical Device Co., Ltd., Ningbo, Zhejiang 315000 (CN)
(72) Inventor: CHEN, Zaihong, Ningbo, Zhejiang 315040 (CN); XUE, Songbiao, Ningbo, Zhejiang 315040 (CN); SHAO, Dingding, Ningbo, Zhejiang 315040 (CN)
(74) Representative: Jell, Friedrich
(86) International application number: PCT/CN2024/098584
(87) International publication number: WO 2025/251332

(57) **Abstract**

Embodiments of the present invention provide an intervention instrument, a control method of an intervention instrument, a computer-readable storage medium and a controller, relating to the technical field of surgical instruments. The control method of an intervention instrument includes: acquiring a position of an end effector of the intervention instrument; and locking or unlocking the end effector according to a relationship between the position where the end effector is located and a pre-set position. By the control method of an intervention instrument, the end effector can be quickly, efficiently and accurately adjusted, so as to realize locking or unlocking.

## Description

### CROSS-REFERENCE TO RELATED APPLICATION

The present invention claims the priority to the Chinese patent application with the filing No. 2024107352804 filed with the Chinese Patent Office on June 7, 2024 and entitled "INTERVENTION INSTRUMENT, CONTROL METHOD OF INTERVENTION INSTRUMENT, STORAGE MEDIUM AND CONTROLLER", the contents of which are incorporated herein by reference in entirety.

### TECHNICAL FIELD

The present invention relates to the technical field of surgical instruments, and specifically to an intervention instrument, a control method of an intervention instrument, a computer-readable storage medium and a controller.

### BACKGROUND ART

An intervention instrument is such an instrument that an end effector needs to be extended into a human body for surgery. A common intervention instrument is generally an endoscopic (laparoscope) instrument, which may include a stapler, a high-frequency electrotome, etc. Generally, the end effector of some intervention instruments can swing relative to a main body unit thereof so as to adjust a position.

Regarding such intervention instruments, since the position is manually adjusted in the prior art, when they need to be adjusted to a pre-set position, adjustment is often inaccurate, thus affecting working efficiency.

### SUMMARY

Embodiments of the present invention provide an intervention instrument, a control method of an intervention instrument, a computer-readable storage medium and a controller, which can realize quick, efficient and accurate adjustment of the end effector so as to realize locking or unlocking.

The embodiments of the present invention can be realized as follows.

Embodiments of the present invention provide a control method of an intervention instrument, which includes:
acquiring a position of an end effector of the intervention instrument; and
locking or unlocking the end effector according to a relationship between the position where the end effector is located and a pre-set position.

Optionally, the pre-set position includes a pre-set pulling-out position of the end effector;
the step of locking or unlocking the end effector according to a relationship between the position where the end effector is located and a pre-set position includes:
locking the end effector in a situation that the end effector moves to the pre-set pulling-out position, so that the end effector remains in the pre-set pulling-out position.

Optionally, the step of acquiring a position of an end effector of the intervention instrument includes:
acquiring a swing angle of the end effector of the intervention instrument relative to a main body unit of the intervention instrument; and
determining, according to the swing angle, the position of the end effector of the intervention instrument.

Optionally, the step of acquiring a position of an end effector of the intervention instrument includes:
acquiring operating data of a drive source; and
determining the position of the end effector according to the operating data,
wherein the drive source is configured to drive the end effector of the intervention instrument to move relative to the main body unit.

Optionally, the step of acquiring a position of an end effector of the intervention instrument includes:
acquiring a position signal representing the end effector output by the position sensor; and
determining the position of the end effector according to the position signal.

Optionally, the step of acquiring a position of an end effector of the intervention instrument includes:
acquiring operating data of a drive source; and determining a first position of the end effector according to the operating data, wherein the drive source is configured to drive the end effector of the intervention instrument to move relative to the main body unit;
acquiring the position signal representing the end effector output by the position sensor; and determining a second position of the end effector according to the position signal; and
determining, in a situation that the first position and the second position are consistent, the first position or the second position as the position of the end effector; and determining, in a situation that the first position and the second position are inconsistent, the position of the end effector according to a pre-set rule.

Optionally, the step of determining the position of the end effector according to a pre-set rule includes:
determining an intermediate position of the first position and the second position as the position of the end effector.

Optionally, the step of locking the end effector includes:
locking the drive source so as to lock the end effector,
wherein the drive source is configured to drive the end effector of the intervention instrument to move relative to the main body unit.

Optionally, after the step of locking the end effector, the control method of an intervention instrument further includes:
unlocking the end effector in a situation that a pre-set unlocking condition is met.

Optionally, the step of unlocking the end effector in a situation that a pre-set unlocking condition is met includes:
unlocking the end effector in a situation that a first trigger signal is acquired,
wherein the first trigger signal is triggered by a control key.

Optionally, the step of unlocking the end effector in a situation that a pre-set unlocking condition is met includes:
unlocking the end effector in a situation that a second trigger signal is acquired,
wherein the second trigger signal represents that under control of a control key, the end effector moves away from a pre-set pulling-out position.

Optionally, the step of unlocking the end effector in a situation that a pre-set unlocking condition is met includes:
unlocking the end effector in a situation that a first trigger-releasing signal is acquired and a third trigger signal is acquired,
wherein the first trigger-releasing signal represents that an action of one of the control keys being triggered to execute controlling the end effector to move towards a pre-set pulling-out position is released, and the third trigger signal represents that another control key is triggered.

Optionally, the step of unlocking the end effector in a situation that a pre-set unlocking condition is met includes:
unlocking the end effector in a situation that a second trigger-releasing signal is acquired and a fourth trigger signal is acquired after first pre-set duration,
wherein the second trigger-releasing signal represents that a control key is triggered by a set action to control the end effector to rotate in a single direction so as to be unlocked; and the fourth trigger signal represents that the control key is triggered again by the set action to control the end effector to rotate in a single direction.

Optionally, the step of unlocking the end effector in a situation that a pre-set unlocking condition is met includes:
unlocking the end effector in a situation that duration of a trigger signal of a control key acquired is longer than or equal to second pre-set duration.

Embodiments of the present invention further provide an intervention instrument, which includes:
a main body unit;
an end effector, wherein the end effector is movable relative to the main body unit;
a drive source, wherein the drive source is configured to drive the end effector of the intervention instrument to move relative to the main body unit; and
a controller, wherein the controller is in communication connection with the drive source, and the controller is configured to implement the above control method of an intervention instrument.

Optionally, the controller is further configured to determine the position of the end effector according to operating data of the drive source; and configured to lock or unlock the end effector according to the relationship between the position where the end effector is located and the pre-set position.

Optionally, the intervention instrument further includes a position sensor, wherein the position sensor is mounted to the main body unit or the end effector; the position sensor is in communication connection with the controller, and the position sensor is configured to detect a position of the end effector relative to the main body unit;
the controller is configured to lock or unlock the end effector according to the relationship between the position where the end effector is located and the pre-set position.

Optionally, the intervention instrument further includes a control key in communication connection with the controller; and
the controller is configured to control the drive source according to a signal of the control key.

Optionally, a plurality of control keys are provided;
at least one of the control keys is distributed on at least one side of the main body unit; or
a plurality of the control keys are distributed on the same side of the main body unit, and among a plurality of the control keys located on the same side, at least one of the control keys is configured to control the end effector to rotate forward, and at least one of the control keys is configured to control the end effector to rotate reversely.

Optionally, the intervention instrument further includes an intermediate transmission unit, and the drive source drives the end effector to move relative to the main body unit through the intermediate transmission unit.

Optionally, the intermediate transmission unit includes a lead-screw mechanism, a traction element, a gear-rack mechanism and a gear assembly, wherein
the lead-screw mechanism includes a screw, a nut and a slider mounted to the main body unit, the drive source is connected to the screw, the screw is in threaded connection with the nut, and the nut is connected to the slider;
the gear-rack mechanism includes a limiting rack and a first gear mounted to the main body unit, wherein the limiting rack is connected to the slider through the traction element, and the limiting rack meshes with the first gear; and
the gear assembly includes a second gear and a joint gear, wherein the second gear is mounted to the main body unit, the second gear meshes with the first gear, the joint gear is mounted to the end effector, and the joint gear meshes with the second gear.

Embodiments of the present invention further provide a computer-readable storage medium, wherein the computer-readable storage medium stores a computer program/instruction, and the computer program/instruction, when executed, implements the above control method of an intervention instrument.

Embodiments of the present invention further provide a controller, including a computer program/instruction, wherein the computer program/instruction, when executed, implements the above control method of an intervention instrument.

The beneficial effects of the embodiments of the present invention include, for example:

The control method of an intervention instrument includes: acquiring the position of the end effector of the intervention instrument; and locking or unlocking the end effector according to the relationship between the position where the end effector is located and the pre-set position. For example, in a situation that the end effector needs to be adjusted to the pre-set position, the end effector may be manually controlled to be adjusted in a normal manner; and in a situation that the end effector is adjusted to the pre-set position, the end effector is locked. Therefore, the adjustment can be quickly, efficiently and accurately realized. For example, after sudden power-off and restart of the intervention instrument, or in a situation of accidental deadlock, it can be judged whether the position where the end effector is located is the pre-set position. In a certain scenario, if the end effector is located in the pre-set position, it indicates that work can be carried out normally, and the end effector can be controlled to be unlocked. If the end effector is not located in the pre-set position, it indicates that manual maintenance is required, and thus the end effector can be controlled to remain locked. In a certain scenario, if the end effector is located in the pre-set position, manual maintenance is required, and thus the end effector can be controlled to remain locked. If the end effector is not located in the pre-set position, it indicates that work can be carried out normally, and the end effector can be controlled to be unlocked.

### BRIEF DESCRIPTION OF DRAWINGS

In order to more clearly illustrate the technical solutions of embodiments of the present invention, the drawings which need to be used in the embodiments will be briefly introduced below. It should be understood that the drawings below merely show some embodiments of the present invention, and thus should not be considered as limitation to the scope. Those ordinarily skilled in the art still could obtain other relevant drawings according to these drawings, without using any inventive efforts.
FIG. 1 is a schematic diagram of an intervention instrument provided in embodiments of the present invention from a first viewing angle;
FIG. 2 is a schematic diagram of the intervention instrument provided in embodiments of the present invention in different states;
FIG. 3 is a schematic diagram of the intervention instrument provided in embodiments of the present invention from a second viewing angle;
FIG. 4 is a partial enlarged schematic diagram of part of structures in FIG. 3;
FIG. 5 is a brief schematic diagram of circuits of various parts of the intervention instrument provided in embodiments of the present invention;
FIG. 6 is a schematic diagram of a control method of an intervention instrument provided in embodiments of the present invention;
FIG. 7 is a schematic diagram of specific steps of S100 shown in FIG. 6;
FIG. 8 is a schematic diagram of specific steps of S200 shown in FIG. 6; and
FIG. 9 is a schematic diagram of specific steps of S300 shown in FIG. 6.

Reference signs: 100-intervention instrument; 10-main body unit; 20-end effector; 30-drive source; 40-intermediate transmission unit; 41-lead-screw mechanism; 411-screw; 412-nut; 413-slider; 42-traction element; 43-gear-rack mechanism; 431-limiting rack; 432-first gear; 44-gear assembly; 441-second gear; 442-joint gear; 50-controller; 60-control key; 61-first control key; 62-second control key; 70-position sensor; 80-computer-readable storage medium.

### DETAILED DESCRIPTION OF EMBODIMENTS

In order to make the objectives, technical solutions and advantages of the embodiments of the present invention clearer, the technical solutions in the embodiments of the present invention will be clearly and completely described with reference to the drawings in the embodiments of the present invention. Apparently, only some but not all embodiments of the present invention are described. Generally, components in the embodiments of the present invention described and shown in the drawings herein may be arranged and designed in different configurations.

Therefore, the following detailed description of the embodiments of the present invention provided in the drawings is not intended to limit the scope of protection of the present invention, but merely represents chosen embodiments of the present invention. Based on the embodiments in the present invention, all of other embodiments obtained by those ordinarily skilled in the art, without using any inventive efforts, shall fall within the scope of protection of the present invention.

It should be noted that like reference signs and letters represent like items in the following drawings, and thus, once a certain item is defined in one drawing, it is unnecessary to define the same in subsequent drawings.

In the description of the present invention, it should be indicated that the orientation or positional relationships indicated by the terms such as "center", "up", "down", "left", "right", "vertical", "horizontal", "inside", and "outside", if appear, are based on the orientation or positional relationships shown in the drawings, or orientation or positional relationships in which the product of the present invention is conventionally placed in use, only for facilitating the description of the present invention and simplifying the description, rather than indicating or implying that the referred devices or elements must be in a particular orientation or constructed or operated in the particular orientation, and thus they should not be construed as limitation to the present invention.

Besides, the terms such as "first", "second", and "third", if appear, are merely used for distinguishing the description, but should not be construed as indicating or implying importance in the relativity.

Moreover, the terms such as "horizontal", "vertical", and "pendulous", if appear, do not mean that a component is required to be absolutely horizontal or pendulous, but mean that the component may be slightly inclined. For example, by "horizontal" it merely means that a structure is more horizontal in comparison with "vertical", rather than being completely horizontal, while the structure may be slightly inclined.

In the description of the present invention, it should also be indicated that unless explicitly otherwise specified and defined, the terms such as "provide", "mount", "link", and "connect", if appear, should be understood in a broad sense, for example, a connection may be a fixed connection, a detachable connection, or an integrated connection; it may be a mechanical connection or an electrical connection; it may be a direct connection or an indirect connection through an intermediate medium, and it may also be internal communication between two elements. For those ordinarily skilled in the art, specific meanings of the above-mentioned terms in the present invention could be understood according to specific circumstances.

It should be noted that features in the embodiments of the present invention may be combined with each other without conflicts.

An intervention instrument is such an instrument that an end effector needs to be extended into a human body for performing surgery. A common intervention instrument is generally an endoscopic instrument, which may include a stapler, a high-frequency electrotome, etc. Generally, the end effector of some intervention instruments can swing relative to a main body unit thereof so as to adjust a position. Regarding such intervention instruments, since the position is manually adjusted in the prior art, when they need to be adjusted to a pre-set position, adjustment is often inaccurate, thus affecting working efficiency.

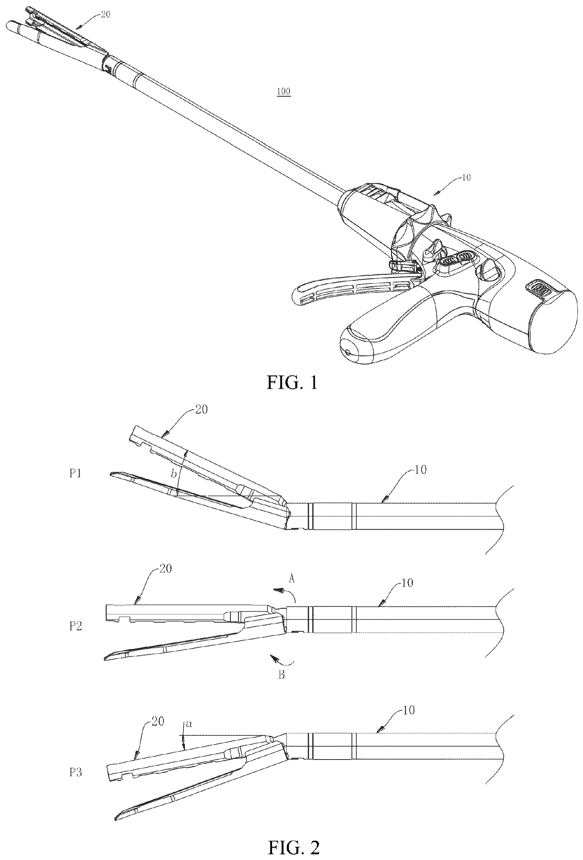

Therefore, referring to FIG. 1 to FIG. 9, embodiments of the present invention provide an intervention instrument 100, a control method of an intervention instrument, a computer-readable storage medium 80 and a controller 50, which can realize quick, efficient and accurate adjustment of an end effector 20 so as to realize locking or unlocking.

They are described in detail below.

Referring to FIG. 1 to FIG. 5, FIG. 1 to FIG. 5 show relevant structural schematic diagrams of the intervention instrument 100 provided in embodiments of the present invention. In the present embodiment, the intervention instrument 100 is illustrated by taking a stapler in an endoscopic instrument as an example. But it should be understood that, without violating the general design concept, it can also be configured to be another instrument in which an end effector needs to be adjusted in position or state, which will not be described in detail subsequently.

Specifically, the intervention instrument 100 includes a main body unit 10, the end effector 20, a drive source 30 and the controller 50.

Generally, in a process of a surgery, a surgeon manipulates a proximal portion of the main body unit 10, and the end effector 20 is connected to a distal portion of the main body unit 10 and is generally configured to be extended into a human body to perform a corresponding operation. The end effector 20 is movable relative to the main body unit 10, so that the position or state of the end effector 20 can be adjusted, and thus the surgeon can manipulate correspondingly. The drive source 30 is configured to drive the end effector 20 of the intervention instrument 100 to move relative to the main body unit 10, and in other words, automatic control may be realized by using the drive source 30.

In the present embodiment, the end effector 20 may include a clamping subunit, a stitching subunit and a cutting subunit, which are not described in detail herein. A motor may be used as the drive source 30, and certainly it is not excluded that a hydraulic motor, a pneumatic motor, an air cylinder, an oil cylinder, etc. are used in specific implementation.

Referring to FIG. 1 and FIG. 2 in combination, in the present embodiment, the end effector 20 as a whole swinging relative to the main body unit 10 is mainly taken as an example for description, that is, a movement manner is rotation, and a rotation angle is smaller than 360 degrees. Specifically, the drive source 30 can drive the end effector 20 to rotate forward or reversely relative to the main body unit 10.

Certainly, in specific implementation, the end effector 20 may also rotate relative to the main body unit 10 by an angle of 360 degrees or more than 360 degrees. Optionally, the end effector 20 may also linearly move relative to the main body unit 10. Optionally, the movement may include both rotation and translation, or spatial movement of multiple degrees of freedom, or irregular movement.

It should be noted that, optionally, a connection position of the end effector 20 relative to the main body unit 10 is relatively fixed, but a posture (attitude) of the end effector 20 itself is changed, so that a position of the end effector 20 as a whole is changed relative to the main body unit 10, which mode may also be understood as that the end effector 20 is movable relative to the main body unit 10.

Referring to FIG. 2, it can be understood that an A direction is a forward-rotation direction, and a B direction is a reverse-rotation direction. For example, FIG. 2 shows that the end effector 20 is in different positions, that is, a P1 position, a P2 position and a P3 position.

If the end effector 20 located in the P1 position rotates forward in the A direction, the position of the end effector 20 below (namely, the P3 position) can be presented. In conjunction with FIG. 2, it can be seen that a forward rotation angle is a, and it can be understood that a swing angle is the angle a.

If the end effector 20 located in the P1 position rotates reversely in the B direction, the position of the end effector 20 above (namely, the P1 position) can be presented. In conjunction with FIG. 2, it can be seen that a reverse rotation angle is b, and it can be understood that a swing angle is the angle b.

It should be noted herein that, description such as forward rotation and reverse rotation are only intended to illustrate relative relationships in conjunction with the drawings, rather than absolutely limiting rotation orientation thereof.

Generally, it can be understood that, when the end effector 20 needs to be pulled out from the human body, it is often necessary to control the end effector 20 to be coaxial and remain locked with the main body unit 10. For example, in conjunction with FIG. 2, the end effector 20 is made to be located in a pre-set pulling-out position, and reference can be made to the P2 position.

The controller 50 is in communication connection with the drive source 30. The controller 50 is configured to realize control over the drive source 30, for example, it can control the drive source 30 to work or stop, control the drive source 30 to perform a corresponding action according to an instruction, and so on. In the present embodiment, the controller 50 is further configured to implement the control method of an intervention instrument.

For example, the controller 50 is configured to acquire the position of the end effector 20 of the intervention instrument 100; and

The end effector 20 is locked or unlocked according to a relationship between the position where the end effector 20 is located and a pre-set position.

Specifically, the pre-set pulling-out position (P2 position) is set as the pre-set position, in this way, when the end effector 20 needs to be pulled out from the human body, the drive source 30 can be manually controlled to work, and when the end effector 20 swings to the pre-set pulling-out position, the controller 50 can control the end effector 20 to be locked, facilitating pulling out the end effector 20.

Certainly, several common positions may also be set as the pre-set position, which may be set according to common types of surgeries or habits of surgeons. In other words, according to normal operations of surgeons, in a situation that the end effector 20 is located in the pre-set position, the end effector 20 will be locked to maintain a current state, which facilitates quick positioning operation by the surgeons.

Certainly, when the intervention instrument 100 is suddenly powered off or accidentally deadlocked in use or test, if it is suddenly restarted, the intervention instrument 100 may also warn, and by judging the relationship between the position where the end effector 20 is located and the pre-set position, it is further judged whether unlocking is needed.

Specifically, this control method of an intervention instrument will be described in detail later.

Certainly, in order to facilitate information input to the controller 50, the intervention instrument 100 further includes a control key 60, wherein the control key 60 is in communication connection with the controller 50; and the controller 50 is configured to control the drive source 30 according to a signal of the control key 60.

For example, the number of the control keys 60 is one, and different signal inputs are given to the controller 50 by pressing or releasing pressing, long-press or short-press, or the like. For example, the drive source 30 works when the control key 60 is pressed, and the drive source 30 stops when the control key 60 is released. For example, when the number of the control keys 60 is two, optionally, one control key 60 is configured to control the drive source 30 to rotate forward, so as to realize the forward rotation of the end effector 20, and the other control key 60 is configured to control the drive source 30 to rotate reversely, so as to realize the reverse rotation of the end effector 20.

In the present embodiment, when the number of the control keys 60 is large, optionally, there is also another control key 60 configured to control the drive source 30 to rotate at different gears. For example, when this control key 60 is pressed once, in a subsequent scenario of controlling the drive source 30, the drive source 30 runs at a low-gear speed, and when this control key 60 is pressed once again, in a subsequent scenario of controlling the drive source 30, the drive source 30 runs at a high-gear speed.

Optionally, the control key 60 may not be presented in a press form. For example, the control key 60 is presented in a form of joystick, and by pulling the joystick towards different directions, the forward rotation or reverse rotation of the drive source 30 are realized. For another example, by twisting the joystick, adjustment of a rotational speed output by the drive source 30 is realized.

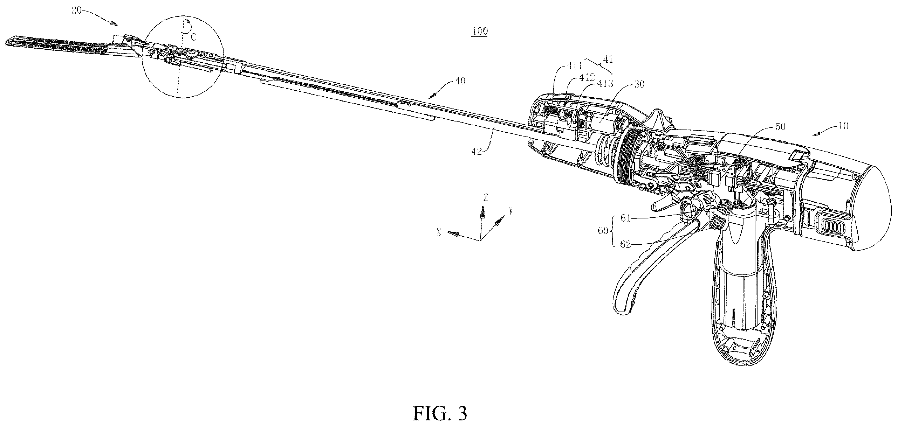

In order to facilitate understanding relevant positional relationships, FIG. 3 shows X, Y and Z directions for auxiliary illustration. In the figure, Z-axis represents a vertical direction, that is, an up-down position, and a positive direction of the Z-axis (that is, a direction pointed by an arrow on the Z-axis) represents up, and a negative direction of the Z-axis represents down. In the figure, X-axis represents a front-back position, and a positive direction of the X-axis (that is, a direction pointed by an arrow on the X-axis) indicates a front side, and a negative direction of the X-axis indicates a rear side. In the figure, Y-axis represents a horizontal direction and indicates a left-right position, a positive direction of the Y-axis (that is, a direction pointed by an arrow on the Y-axis) represents a right side, and a negative direction of the Y-axis represents a left side. Meanwhile, it should be noted that meanings represented by the foregoing Z-axis, Y-axis and X-axis are merely for facilitating describing the present embodiment and simplifying the description, rather than indicating or implying that related devices or elements have to be in the specific orientation or configured and operated in a specific orientation, and thus should not be construed as limitation to the present invention.

Optionally, referring to FIG. 3 to FIG. 5 in combination, there are a plurality of control keys 60, and "a plurality of" herein may be construed as being greater than or equal to two. Based on this, optionally, at least one control key 60 is distributed on at least one side of the main body unit 10, in other words, one side of the main body unit 10 may be provided with one or more control keys 60, or both sides of the main body unit 10 may be provided with one or more control keys 60.

Optionally, a plurality of control keys 60 are distributed on the same side of the main body unit 10, and among the plurality of control keys 60 located on the same side, at least one control key 60 is configured to control the end effector 20 to rotate forward, and at least one control key 60 is configured to control the end effector 20 to rotate reversely.

For example, in a scenario of manual manipulation, at least one control key 60 located on a left side of the main body unit 10 is configured to control the drive source 30 to rotate forward, so as to realize the forward rotation of the end effector 20, and at least one control key 60 located on the left side of the main body unit 10 is configured to control the drive source 30 to rotate reversely, so as to realize the reverse rotation of the end effector 20.

Specifically, the plurality of control keys 60 include a first control key 61 and a second control key 62 located on one side of the main body unit 10. Herein, the first control key 61 is configured to control the drive source 30 to rotate forward, so as to realize the forward rotation of the end effector 20, and the second control key 62 is configured to control the drive source 30 to rotate reversely, so as to realize the reverse rotation of the end effector 20.

Certainly, it is not excluded that in a certain embodiment, different control keys 60 located on both left and right sides of the main body unit 10 are configured to control the drive source 30 to rotate forward and reversely, respectively.

Therefore, in combination with the above, it can be seen that the control keys 60 may be switch keys, speed-regulating keys, direction keys, etc.

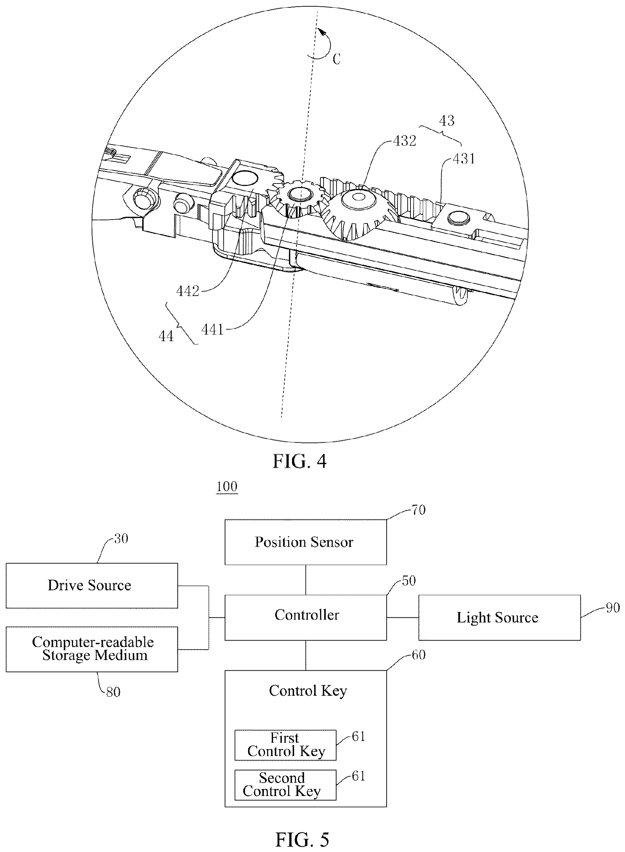

Referring to FIG. 3 and FIG. 4 in combination, in the present embodiment, the intervention instrument 100 further includes an intermediate transmission unit 40, and the drive source 30 drives the end effector 20 to move relative to the main body unit 10 through the intermediate transmission unit 40. In other words, the drive source 30 does not directly drive the end effectors 20, but instead indirectly.

For example, the intermediate transmission unit 40 includes a speed reducer, by which speed reduction is realized. For example, the intermediate transmission unit 40 includes various mechanisms, and long-distance driving is realized by a rod-linkage mechanism, a worm-gear mechanism, a sprocket-chain mechanism, a belt-pulley mechanism, etc. Certainly, it is not excluded that in specific implementation, the drive source 30 is mounted to the main body unit 10 and directly drives the end effector 20 to move relative to the main body unit 10. Alternatively, the drive source 30 is mounted to the end effector 20 and directly drives the end effector 20 to move relative to the main body unit 10.

In the present embodiment, the intermediate transmission unit 40 includes a lead-screw mechanism 41, a traction element 42, a gear-rack mechanism 43 and a gear assembly 44.

The lead-screw mechanism 41 includes a screw 411, a nut 412 and a slider 413 mounted to the main body unit 10, the drive source 30 is connected to the screw 411, the screw 411 is in threaded connection with the nut 412, and the nut 412 is connected to the slider 413.

The gear-rack mechanism 43 includes a limiting rack 431 and a first gear 432 mounted to the main body unit 10, wherein the limiting rack 431 is connected to the slider 413 through the traction element 42, and the limiting rack 431 meshes with the first gear 432.

The gear assembly 44 includes a second gear 441 and a joint gear 442, wherein the second gear 441 is mounted to the main body unit 10, the second gear 441 meshes with the first gear 432, the joint gear 442 is mounted to the end effector 20, and the joint gear 442 meshes with the second gear 441.

Specifically, the drive source 30 directly drives the screw 411 to rotate, and rotation of the screw 411 drives the nut 412 to linearly move, thereby driving the slider 413 to linearly move in the front-back direction. Since the slider 413 is connected to the traction element 42 (for example, a pull tab), the traction element 42 is also driven to linearly move in the front-back direction. The traction element 42 is connected to the limiting rack 431, and the limiting rack 431 meshes with the first gear 432; therefore, during movement of the traction element 42, the limiting rack 431 is driven to move, further realizing rotation of the first gear 432. Since the first gear 432 meshes with the second gear 441, rotation of the second gear 441 is realized. The joint gear 442 is fixed relative to the end effector, and since the joint gear 442 meshes with the second gear 441, the second gear 441 forcedly drives the joint gear 442 to rotate, and finally drives the end effector 20 to rotate (namely, swing) relative to the main body unit 10.

Optionally, a diameter of the first gear 432 is larger than that of the second gear 441. Certainly, in specific implementation, a relationship between diameters of the first gear 432, the second gear 441 and the joint gear 442 is not limited.

Optionally, an axis of rotation of the end effector relative to the main body unit 10 is coaxial with an axis of rotation of the second gear 441, that is, both the axes are axis C in FIG. 4. In this way, running stability is strong.

It should be noted that, referring to FIG. 2 and FIG. 4 in combination, the end effector 20 may rotate about the axis C relative to the main body unit 10. It can be understood that, if the end effector 20 rotates in a direction indicated by the arrow, it is in forward rotation; if the end effector 20 rotates in a direction opposite to the direction indicated by the arrow, it is in reverse rotation, so that the end effector 20 swings about this axis C.

In the present embodiment, the controller 50 can be used to realize locking of the end effector 20 to a desired position or unlocking. For example:

The controller 50 is further configured to determine the position of the end effector 20 according to operating data of the drive source 30; and configured to lock or unlock the end effector 20 according to the relationship between the position where the end effector 20 is located and the pre-set position.

For example, referring to FIG. 2, when the end effector 20 needs to be locked to remain in the P1 position, the P1 position may be set as this pre-set position, and the position of the end effector 20 may be determined by using the operating data of the drive source 30. When the end effector 20 swings in a normal swinging process, once it swings to this pre-set position, the controller 50 immediately controls the end effector 20 to stop swinging and locks it to remain in this position.

In the above, the operating data may include drive data, encoder data, and the like, which data can reflect an angle by which the end effector 20 rotates, and thus can reflect the position of the end effector 20.

Optionally, the locking is realized in a manner of controlling the drive source 30 to stop. For example, the locking of the end effector 20 is realized by applying a brake to the drive source 30. In combination with the above, after the drive source 30 stops running, a locking function may be realized by using the drive source 30 per se, for example, a stepping motor provided with a stop locking function is adopted as the drive source 30. Or, the locking of the end effector 20 is realized by a self-lock function of the lead-screw mechanism 41 in the intermediate transmission unit 40. Or, a locking structure is separately designed, and it is installed near the drive source 30, or is directly installed on any component of the intermediate transmission unit 40, and by clamping and fixing the any component, the locking of the end effector 20 is realized.

Likewise, the P2 position, the P3 position, or any position may be set as this pre-set position. For example, several positions commonly used in surgeries may be set as the pre-set position. When the P2 position is chosen as the pre-set position, this position may also be used as a pulling-out position of the end effector 20, that is, a relatively preferred position where it is pulled out of the human body.

It should be noted that, several positions mentioned above may be directly set as the pre-set position, or only one position may be set as the pre-set position. Through one of the control keys 60, a certain angle may be increased or decreased on the basis of the pre-set position, and in this way, when reaching a position obtained by increasing or decreasing a certain angle on the basis of this pre-set position, locking of the end effector 20 is realized.

For example, in conjunction with FIG. 2, the P2 position may be set as the pre-set position, and one of the control keys 60 is used to increase, for example, 30 degrees, so that the end effector 20 will be locked in the P3 position, and in this case, in the P3 position, the end effector 20 rotates by 30 degrees relative to the P2 position.

Certainly, a sensor may also be used to directly detect the position, for example, the intervention instrument 100 further includes a position sensor 70, wherein the position sensor 70 is mounted to the main body unit 10 or the end effector 20. The position sensor 70 is in communication connection with the controller 50, and the position sensor 70 is configured to detect the position of the end effector 20 relative to the main body unit 10. Likewise, the controller 50 is configured to lock or unlock the end effector 20 according to the relationship between the position where the end effector 20 is located and the pre-set position.

Certainly, it should be noted that the number of position sensors 70 is not limited, for example, the number of position sensors 70 may be two, and data detected by the two position sensors 70 are compared. When two detected positions are the same, a result output by one of the position sensors 70 is selected. If the positions detected by the two position sensors 70 are different and deviation is within a pre-set range, an intermediate position of the positions detected by the two position sensors 70 may be taken as characterizing the position of the end effector 20 relative to the main body unit 10. If the positions detected by the two position sensors 70 are different and deviation is outside the pre-set range, it may be identified that at least one position sensor 70 fails, and shutdown maintenance is required.

Optionally, in the present embodiment, the end effector 20 is provided in a manner of swinging relative to the main body unit 10, and thus an angle sensor may be chosen as the position sensor 70, and configured to detect a swing angle of the end effector 20 relative to the main body unit 10, so as to determine the position of the end effector 20 relative to the main body unit 10.

Certainly, a distance sensor may also be chosen as the position sensor 70, and for example, configured to detect a distance between one or more points on the end effector 20 and one or more points on the main body unit 10, so as to determine the position of the end effector 20 relative to the main body unit 10.

It should be noted that, the position sensor 70 may be used to directly detect the position of the end effector 20 relative to the main body unit 10, and may also detect a position of the intermediate transmission unit 40, so as to obtain the position of the end effector 20. In other words, the position sensor 70 may be mounted to any component of the intermediate transmission unit 40, and in this way, it facilitates position arrangement of the position sensor 70, and can reduce an end dimension of the intervention instrument 100. For example, the traction element 42 is provided thereon with a plurality of trigger points, and when the position sensor 70 is triggered by different trigger points, signals indicating that the end effector 20 is in different positions may be output.

In addition, it is not excluded that the position sensor 70 directly detects a coordinate position of one or more points on the end effector 20 relative to a coordinate system established by the main body unit 10, and the position of the end effector 20 relative to the main body unit 10 is determined by using the coordinate position.

Optionally, X development may be used for position determination, for example, after X development, the position of the end effector 20 relative to the main body unit 10 is directly determined through image recognition. Specifically, it may be realized by providing one or more developing points or developing areas on the end effector 20 and the main body unit 10.

Detection of the angle sensor is taken as an example for description. Generally, for a disposable instrument or an instrument that is rarely used, it is generally identified that when a detected rotation angle of the end effector 20 relative to the main body unit 10 is 0 degrees, it is considered that the end effector 20 reaches the pre-set pulling-out position (for example, the P2 position in FIG. 2).

Certainly, for some types of instruments which may be used for a long time, it is not excluded that the detected angle and an actual angle are not the same, and in a situation that it can be known that the two are inconsistent by visual observation or detection of other tools, fine tuning may be performed by a program. For example, when the detected angle is 0 degrees and the actual angle is 5 degrees, the pre-set angle 0 degrees corresponding to the pre-set position may be adjusted to be -5 degrees.

Referring to FIG. 5, the present embodiment further provides a computer-readable storage medium 80, wherein the computer-readable storage medium 80 stores a computer program/instruction, and when the computer program/instruction is executed, the above control method of an intervention instrument is implemented.

The computer-readable storage medium 80 may be part of the intervention instrument 100, and may also be a separate structure. Optionally, the computer-readable storage medium 80 is fixedly mounted in the main body unit 10 in a fixed manner, and it is in communication connection with the controller 50. The controller 50 is configured to call the computer program/instruction stored in the computer-readable storage medium 80, so as to implement the control method of an intervention instrument.

Certainly, the computer-readable storage medium 80 may also be detachably assembled with the main body unit 10. For example, the main body unit 10 is provided with a clamping groove, and the computer-readable storage medium 80 is clamped in the clamping groove, so as to implement communication with the controller 50 built in the main body unit 10. Optionally, the computer-readable storage medium 80 may be a solid-state memory, a memory card, an optical disc, or the like.

Referring to FIG. 5, it should be noted that the present embodiment further provides a controller 50, including a computer program/instruction, and when the computer program/instruction is executed, the above control method of an intervention instrument is implemented.

The controller 50 is generally built in the main body unit 10. When the computer-readable storage medium 80 is also built in the main body unit 10, it can be understood that the computer-readable storage medium 80 and the controller 50 may be two independent electrical components, and certainly, the two may also be integrated into one.

Referring to FIG. 5, the intervention instrument 100 further includes a light source that is electrically connected to the controller 50.

The light source may emit light when the drive source 30 works. For example, in a situation that the drive source 30 rotates forward or reversely, light of different colors is emitted, or in a situation that the drive source 30 is locked, flickering light, or the like, is emitted.

The intervention instrument 100 may further include a power source configured to supply power to the various electrical components above. Certainly, the intervention instrument 100 may further be powered by an external electrical network through a wire.

Generally, the controller 50 may be integrated on a circuit board, and the circuit board is provided with various ports/interfaces, so as to realize communication with various electrical components by cables.

It should be noted that the control method of an intervention instrument provided in the present embodiment may be implemented in hardware and firmware, or may be software or computer code that can be stored in a computer-readable storage medium, for example, a read- only memory (ROM), a random access memory (RAM), a floppy disk, a hard disk, or a magnetic disk, or may be computer code originally stored on a remote recording medium or a non-transitory machine-readable medium, downloaded over a network and stored in a local recording medium, and thus the method described herein may be embodied in software stored on a recording medium using a general purpose computer or a special processor or in programmable or dedicated hardware, such as an application specific integrated circuit (ASIC) or a field programmable gate array (FPGA). As can be appreciated in the art, the computer, processor, microprocessor, controller or programmable hardware include memory components, such as RAM, ROM, flash memory, etc., and when the computer, processor or hardware implements a processing method described herein to access and execute the software or computer code, the memory components may store or receive the software or computer code. Additionally, when the general purpose computer access is configured to implement the code of processing illustrated herein, the execution of the code transforms the general purpose computer into a special purpose computer configured to perform the processing illustrated herein.

If it is realized in a form of software functional module and sold or used as an independent product, it may be stored in a computer-readable storage medium. Based on such understanding, the above technology can be embodied in a form of software product, and this computer software product is stored in a computer-readable storage medium, including several instructions for making the controller execute all or part of the method steps of the above method.

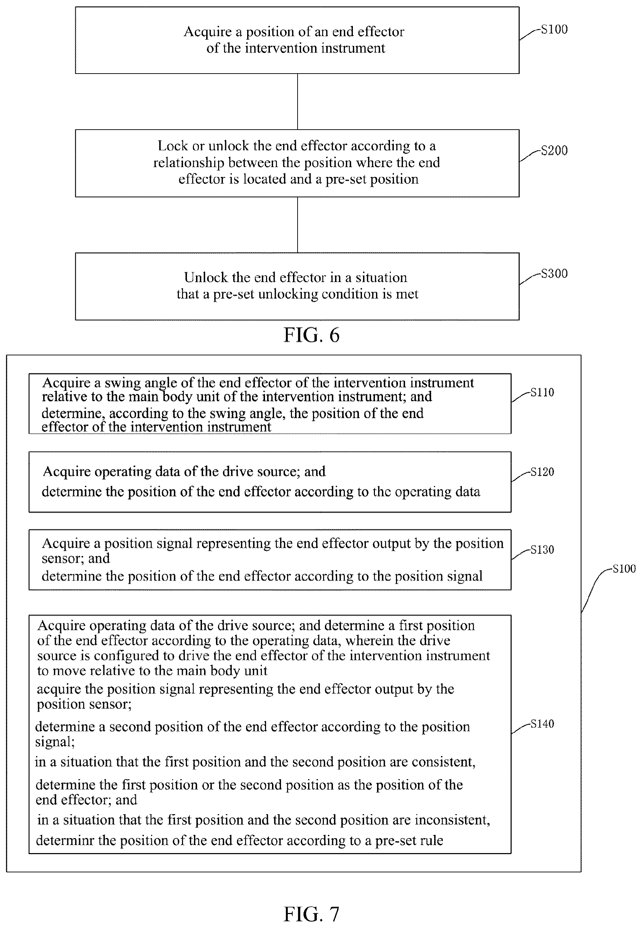

Each block in flowcharts or block diagrams shown in FIG. 6 to FIG. 9 may represent one module, program segment or a part of code, and the module, program segment or a part of code contains one or more executable instructions configured to achieve a specified logical function. It should also be noted that in some implementation modes as substitutions, functions marked in the blocks can also take effect in an order different than that marked in the drawings. For example, two continuous blocks can be executed in parallel or in a reverse order, which depends on the function involved. It should also be noted that each block or combination of the blocks in the block diagrams and/or the flowcharts can be realized by a dedicated hardware-based system executing a specified function or action, or can be realized by a combination of dedicated hardware and computer instructions.

Referring to FIG. 6 to FIG. 9, the present embodiment provides a control method of an intervention instrument. For a specific structure of the intervention instrument 100, reference may be made to relevant structures shown in FIG. 1 to FIG. 5, and details are not described herein. Certainly, reference may also be made to relevant instruments commonly seen in the prior art.

Referring to FIG. 6, specifically, the control method of an intervention instrument includes:
S100: acquiring a position of an end effector 20 of the intervention instrument 100; and
S200: locking or unlocking the end effector 20 according to a relationship between the position where the end effector 20 is located and a pre-set position.

For example, in a situation that the end effector 20 needs to be adjusted to the pre-set position, the end effector 20 may be manually controlled to be adjusted in a normal manner; and in a situation that the end effector 20 is adjusted to the pre-set position, the end effector 20 is locked. Therefore, adjustment can be quickly, efficiently and accurately realized. For example, after sudden power-off and restart of the intervention instrument 100, or in a situation of accidental deadlock, it can be judged whether the position where the end effector 20 is located is the pre-set position.

In a certain scenario, if the end effector 20 is located in the pre-set position, it indicates that work can be carried out normally, and the end effector 20 can be controlled to be unlocked. If the end effector 20 is not located in the pre-set position, it indicates that manual maintenance is required, and thus the end effector 20 can be controlled to remain locked.

In a certain scenario, if the end effector 20 is located in the pre-set position, manual maintenance is required, and thus the end effector 20 can be controlled to remain locked. If the end effector 20 is not located in the pre-set position, it indicates that work can be carried out normally, and the end effector 20 can be controlled to be unlocked.

During a surgery, the end effector 20 of the intervention instrument 100 needs to precisely match a lesion, facilitating performing a corresponding operation (such as anastomosis) through the end effector 20 subsequently. The surgeon may manually press a corresponding control key 60 to make an angle of the end effector 20 relative to the main body unit 10 changed, so as to realize adjustment of the position of the end effector 20. However, after a corresponding operation is completed, when the end effector 20 needs to be pulled out of the human body, difficulties are often encountered, for example, referring to FIG. 2, if the end effector 20 is located in the P1 or P3 position, to directly perform a pulling-out action in this case may have certain safety hazards.

For this case, the surgeon often manually controls the control key 60 so that the end effector 20 is adjusted to be in a state of being coaxial with the main body unit 10 as much as possible (such as the P2 position in FIG. 2). However, manual adjustment often has control errors, for example, a swing angle being insufficient or excessive. Specifically, when adjusting the position of the end effector 20, there is generally no corresponding limit at a pre-set pulling-out position, and the end effector 20 tends to swing excessively in a swinging process and miss the pre-set pulling-out position, which causes pulling-out difficulty and even causes safety hazards.

In order to solve this technical problem, in the present embodiment, optionally, the pre-set position includes the pre-set pulling-out position of the end effector 20.

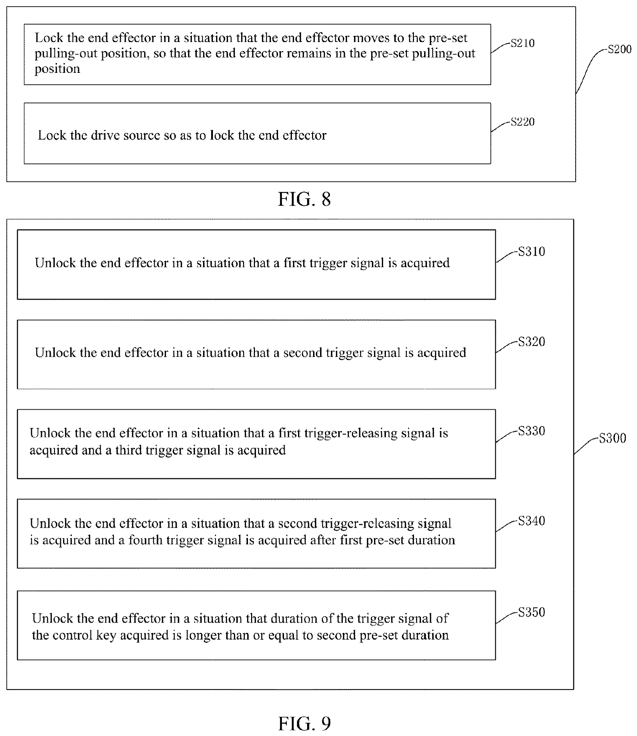

Referring to FIG. 6 and FIG. 8 in combination, the step of locking or unlocking the end effector 20 according to a relationship between the position where the end effector 20 is located and a pre-set position mentioned in the above includes:
S210: locking the end effector 20 in a situation that the end effector 20 moves to the pre-set pulling-out position, so that the end effector 20 remains in the pre-set pulling-out position.

Specifically, in a process that the surgeon manually operates the control key 60 to control the end effector 20 to swing from the P1 and P3 positions towards the P2 position, when the end effector 20 swings to the P2 position, the controller 50 locks the end effector 20, for example, locks a drive source 30, so that the end effector 20 remains in the pre-set pulling-out position. In this case, space required for the process of pulling out the end effector 20 is small, which facilitates the pull-out of the end effector 20, and can meanwhile avoid damage to the human body during the pull-out as much as possible.

Therefore, by acquiring the position where the end effector 20 swings, it can further be judged whether the end effector 20 swings to the pre-set pulling-out position according to the position where the end effector 20 swings. In a situation that the end effector 20 swings to the pre-set pulling-out position, for example, the end effector 20 swings to be coaxial with the main body unit 10, the end effector 20 is controlled to be locked, so that the end effector 20 cannot continue to swing, and the end effector 20 remains in the pre-set pulling-out position, so that a case that the end effector 20 swings excessively and misses the pre-set pulling-out position can be avoided. By means of this method, accuracy of the pulling-out position can be improved, difficulty of manual control can be reduced, and occurrence of collision when pulling out the end effector 20 can be avoided.

It should be noted that, the pre-set position may not only include the pre-set pulling-out position. For example, several positions commonly used by surgeons may also be set as the pre-set position, so as to facilitate accurate positioning of the end effector 20.

Referring to FIG. 6 and FIG. 7 in combination, in the present embodiment, optionally, the step of acquiring a position of an end effector 20 of the intervention instrument 100 includes:
S110: acquiring a swing angle of the end effector 20 of the intervention instrument 100 relative to the main body unit 10 of the intervention instrument 100; and determining, according to the swing angle, the position of the end effector 20 of the intervention instrument 100.

For example, the swing angle is directly detected by a position sensor 70 (e.g., an angle sensor), or the swing angle is obtained by using operating data of the drive source 30. Certainly, it is not excluded that the swing angle is obtained by using X development technology.

It should be noted that, the above end effector 20 is provided in a manner of rotating relative to the main body unit 10, and thus the position of the end effector 20 can be obtained by using a rotation angle (the swing angle). Certainly, by detecting a position of an intermediate transmission unit 40, for example, translational positions of a traction element 42, a nut 412, a slider 413, etc., the position of the end effector 20 can also be indirectly obtained.

Certainly, if the end effector 20 can translate relative to the main body unit 10, the position of the end effector 20 can be obtained by detecting a distance of translation.

Referring to FIG. 6 and FIG. 7 in combination, in the present embodiment, optionally, the step of acquiring a position of an end effector 20 of the intervention instrument 100 includes:
S120: acquiring operating data of the drive source 30; and determining the position of the end effector 20 according to the operating data,
wherein the drive source 30 is configured to drive the end effector 20 of the intervention instrument 100 to move relative to the main body unit 10.

The operating data include drive data, encoder data, and the like. The data such as the drive data and encoder data can reflect an angle by which the end effector 20 rotates, and thus can reflect the position of the end effector 20.

Referring to FIG. 6 and FIG. 7 in combination, in the present embodiment, optionally, the step of acquiring a position of an end effector 20 of the intervention instrument 100 includes:
S130: acquiring a position signal representing the end effector 20 output by the position sensor 70; and determining the position of the end effector 20 according to the position signal.

In conjunction with the above contents, the angle sensor, a distance sensor, etc. can be used as the position sensor 70. The position of the end effector 20 can be determined by detecting a rotation angle of a screw 411, a moving distance of the nut 412, the slider 413, the traction element 42, and a limiting rack 431, or a rotation angle of a first gear 432, a second gear 441, etc.

Referring to FIG. 6 and FIG. 7 in combination, in the present embodiment, optionally, the step of acquiring a position of an end effector 20 of the intervention instrument 100 includes:
S140: acquiring operating data of the drive source 30; and determining a first position of the end effector 20 according to the operating data, wherein the drive source 30 is configured to drive the end effector 20 of the intervention instrument 100 to move relative to the main body unit 10;
acquiring the position signal representing the end effector 20 output by the position sensor 70; and determining a second position of the end effector 20 according to the position signal; and
determining, in a situation that the first position and the second position are consistent, the first position or the second position as the position of the end effector 20; and determining, in a situation that the first position and the second position are inconsistent, the position of the end effector 20 according to a pre-set rule.

In order to improve the accuracy of position detection, the operating data of the drive source 30 and the data detected by the position sensor 70 are compared, wherein in a situation that the two pieces of data are consistent, one of them is selected; and in a situation that the two pieces of data are inconsistent, the position is determined according to the pre-set rule.

In the present embodiment, optionally, the step of determining the position of the end effector 20 according to a pre-set rule includes:
determining an intermediate position of the first position and the second position as the position of the end effector 20.

For example, referring to FIG. 2, assuming that the data detected by the drive source 30 represent that the end effector 20 is located in the P1 position, while the data detected by the position sensor 70 represent that the end effector 20 is located in the P3 position, then the end effector 20 can be roughly determined to be located in the P2 position in this case by calculation.

Certainly, if a difference between the two pieces of detection data is large, the surgeon may judge, according to experience, that there is a problem with the detection data of the position sensor 70 and/or the operating data of the drive source 30 in this case, and further judge, according to severity of the problem, whether shutdown maintenance is required.

In order to facilitate locking the end effector 20, referring to FIG. 6 and FIG. 8 in combination, in the present embodiment, optionally, the step of locking the end effector 20 includes:
S220: locking the drive source 30 so as to lock the end effector 20,
wherein the drive source 30 is configured to drive the end effector 20 of the intervention instrument 100 to move relative to the main body unit 10.

For example, the locking of the end effector 20 can be realized by applying a brake to the drive source 30. In combination with the above, after the drive source 30 stops running, a locking function may be realized by the drive source 30 per se, for example, a stepping motor provided with a stop locking function is adopted as the drive source 30. Or, the locking of the end effector 20 is realized by a self-lock function of a lead-screw mechanism 41 in the intermediate transmission unit 40. Or, a locking structure is separately designed, and it is installed near the drive source 30, or is directly installed on any component of the intermediate transmission unit 40, and by clamping and fixing any component, the locking of the end effector 20 is realized.

Referring to FIG. 6 and FIG. 9 in combination, in the present embodiment, optionally, after the step of locking the end effector 20, the control method of an intervention instrument further includes:
S300: unlocking the end effector 20 in a situation that a pre-set unlocking condition is met.

For example, in a locked state, the pre-set unlocking condition may include: the surgeon triggering the control key 60, or other operations, such as rotating, extending, retracting into the main body unit 10, etc., or sudden power-off, etc. After the pre-set unlocking condition is met, the locking can be released, so that the end effector 20 can continue to be controlled, that is, the end effector 20 is made to be in an unlocked state.

It should be noted that, the control key 60 herein may be a switch button, and may also be other keys that communicate with the controller 50. In combination with the above, it can be seen that the control key 60 may also be a speed-regulating key, a direction key, etc.

In a certain scenario, when a pulling-out operation needs to be performed on the end effector 20, but the end effector 20 is located in a pre-set position other than the pre-set pulling-out position, the surgeon may unlock the end effector 20 in a manner of, for example, triggering the control key 60, so that it continues to swing, and further can swing to the pre-set pulling-out position. Alternatively, after the end effector 20 is located in the pre-set pulling-out position, the surgeon judges that the surgery needs to be continued in this case, thus can trigger the control key 60, so that it continues to swing, proceed to the next step, etc.

Specifically, the pre-set unlocking condition may be set according to actual requirements, which will be exemplarily described subsequently.

Referring to FIG. 6 and FIG. 9 in combination, in the present embodiment, optionally, the step of unlocking the end effector 20 in a situation that a pre-set unlocking condition is met includes:
S310: unlocking the end effector 20 in a situation that a first trigger signal is acquired,
wherein the first trigger signal is triggered by the control key 60.

In other words, the end effector 20 can be unlocked as long as the surgeon triggers the control key 60.

Referring to FIG. 6 and FIG. 9 in combination, in the present embodiment, optionally, the step of unlocking the end effector 20 in a situation that a pre-set unlocking condition is met includes:
S320: unlocking the end effector 20 in a situation that a second trigger signal is acquired,
wherein the second trigger signal represents that under control of the control key 60, the end effector 20 moves away from the pre-set pulling-out position.

In a certain scenario, the control key 60 includes a first control key 61 and a second control key 62, wherein the first control key 61 implements forward rotation of the end effector 20 and the second control key 62 implements reverse rotation of the end effector 20. It can be understood that, the surgeon first operates the first control key 61 to make the end effector 20 move towards the pre-set pulling-out position and finally locked; and when the surgeon triggers the second control key 62, the locking of the end effector 20 is released and unlocking is realized.

Certainly, it is not excluded that in a certain scenario, the surgeon first operates the first control key 61 to make the end effector 20 move towards the pre-set pulling-out position and finally locked; and when the surgeon continues to trigger the first control key 61, the locking of the end effector 20 is released and unlocking is realized.

Alternatively, it is not excluded that in a certain scenario, the surgeon first operates the first control key 61 to make the end effector 20 move towards the pre-set pulling-out position and to be finally locked; and when the surgeon simultaneously triggers the first control key 61 and the second control key 62, the locking of the end effector 20 can be released and unlocking is realized.

Referring to FIG. 6 and FIG. 9 in combination, in the present embodiment, optionally, the step of unlocking the end effector 20 in a situation that a pre-set unlocking condition is met includes:
S330: unlocking the end effector 20 in a situation that a first trigger-releasing signal is acquired and a third trigger signal is acquired,
wherein the first trigger-releasing signal represents that an action of one of the control keys 60 being triggered to execute controlling the end effector 20 to move towards the pre-set pulling-out position is released, and the third trigger signal represents that another control key 60 is triggered.

In other words, in a certain scenario, the control key 60 includes a first control key 61 and a second control key 62, wherein the first control key 61 implements forward rotation of the end effector 20, and the second control key 62 implements reverse rotation of the end effector 20. It can be understood that, the surgeon first operates the first control key 61 to make the end effector 20 move towards the pre-set pulling-out position and finally locked; when the surgeon releases the first control key 61, the first trigger-releasing signal is output, and when the surgeon triggers the second control key 62, the locking of the end effector 20 is released and unlocking is realized.

Certainly, it is not excluded that in a certain scenario, the surgeon first operates the first control key 61 to make the end effector 20 move towards the pre-set pulling-out position and finally locked; when the surgeon releases the first control key 61, the first trigger-releasing signal is output, and when the surgeon triggers another control key 60, the locking of the end effector 20 is released and unlocking is realized.

It can be seen from specific structures of the intervention instrument 100 described in the preceding that, in a situation that the number of the control keys 60 is plural, the plurality of control keys 60 may be distributed on one side of the main body unit 10, and it is also feasible that the control keys 60 are distributed on both sides of the main body unit 10. In addition, all functions of the plurality of control keys 60 may be inconsistent, or functions of some of the control keys 60 are consistent. Therefore, "another control key 60" mentioned in the above can be understood as a control key 60 that does not perform preceding operations.

Optionally, both left and right sides of the main body unit 10 are provided with a group of control keys 60 respectively, and each group of control keys 60 include the first control key 61 and second control key 62 in the above. The "another control key 60" mentioned herein may be control key 60 with another function on the same side, and may also be control key 60 with the same or different function on a different side. By controlling the end effector 20 on both left and right sides respectively, use requirements of different populations, such as left-handers and right-handers, can be met, and operators can switch hands to operate, thus reducing fatigue during operation.

Referring to FIG. 6 and FIG. 9 in combination, in the present embodiment, optionally, the step of unlocking the end effector 20 in a situation that a pre-set unlocking condition is met includes:
S340: unlocking the end effector 20 in a situation that a second trigger-releasing signal is acquired and a fourth trigger signal is acquired after a first pre-set duration,
wherein the second trigger-releasing signal represents that the control key 60 is triggered by a set action to control the end effector 20 to rotate in a single direction so as to be unlocked; and the fourth trigger signal represents that the control key 60 is triggered again by the set action to control the end effector 20 to rotate in a single direction.

The first pre-set duration can be set according to specific conditions, for example, the first pre-set duration may be set to be 0.5 s, 1 s, 1.5 s, 3 s, and so on.

In other words, in a certain scenario, the control key 60 includes a first control key 61 and a second control key 62, wherein the first control key 61 implements forward rotation of the end effector 20, and the second control key 62 implements reverse rotation of the end effector 20. It can be understood that, the surgeon first operates the first control key 61 to make the end effector 20 move towards the pre-set pulling-out position and finally locked; when the surgeon releases the first control key 61, the second trigger-releasing signal is output, and after the first pre-set duration, the surgeon triggers the first control key 61 so as to output the fourth trigger signal, and the locking of the end effector 20 is released and unlocking is realized.

The set action is determined according to actual conditions of the control key 60, for example, if the control key 60 is a press key, the set action is an action of pressing the control key 60. For example, if the control key 60 is a push key, the set action is an action of pushing the control key 60.

The above description mainly takes the control keys 60 being press keys as an example. Specifically, a press key is released first, and then the same press key is pressed again.

It should be understood that, according to a general triggering manner of the control key 60, continuous control is usually implemented by keeping triggering the control key 60, i.e., keeping the set action to trigger the control key 60, that is, controlling the end effector 20 to rotate in a single direction, for example, rotate forward, rather than releasing the control key 60 and pressing the control key 60 again. In this way, this arrangement manner can avoid a case that the end effector 20 rotates excessively and misses the pre-set pulling-out position due to too long time of triggering the control key 60 by the set action, can avoid a case that the end effector 20 rotates excessively and misses the pre-set pulling-out position due to inaccurate control over action duration of the set action, and can improve position stability of the end effector 20 in the pre-set pulling-out position.

Certainly, it is not excluded that in a certain scenario, the surgeon first operates the first control key 61 to make the end effector 20 move towards the pre-set pulling-out position and finally locked; and when the surgeon releases the first control key 61, the second trigger-releasing signal is output, and after the first pre-set duration, when the surgeon triggers the second control key 62, the fourth trigger signal is output, and the locking of the end effector 20 is released and unlocking is realized.

Referring to FIG. 6 and FIG. 9 in combination, in the present embodiment, optionally, the step of unlocking the end effector 20 in a situation that a pre-set unlocking condition is met includes:
S350: unlocking the end effector 20 in a situation that duration of the trigger signal of the control key 60 acquired is longer than or equal to a second pre-set duration.

In other words, in a situation that the end effector 20 is locked, if the surgeon continuously presses the control key 60 for the second pre-set duration, the locking of the end effector 20 is released, and unlocking is realized. The second pre-set duration may be set according to specific conditions, for example, the second pre-set duration may be set to be 1 s, 2 s, 3 s, 4 s, and so on. With the duration, accidental unlocking of the end effector 20 caused by the surgeon's unintended trigger can be avoided.

Optionally, the second pre-set duration is greater than the first pre-set duration, for example, the second pre-set duration may be M times the first pre-set duration, M is greater than 1, for example, M may be 2, 3, 4, and so on.

It can be understood that, when the duration for which the control key 60 is continuously triggered is longer than or equal to the second pre-set duration, it indicates that the operator does not intend to control the end effector 20 to swing to the pre-set pulling-out position and remain in the pre-set pulling-out position, and his intention of triggering the control key 60 is to swing the end effector 20 from one side to the other side through the pre-set pulling-out position, and in this case, the position of the end effector 20 is unlocked, and the end effector 20 can be controlled to execute an actual control intention, which unlocking idea is more humanized.

In combination with the above, for various unlocking conditions mentioned above, it can be understood that the unlocking can be realized when any unlocking condition therein is met. In the present embodiment, five unlocking conditions in total for S310, S320, S330, S340, S350 and the like are shown.

In specific implementation, the control method of an intervention instrument further includes: controlling, when a control-mode instruction is received, the end effector 20 according to a control mode.

For example, the control-mode instruction includes one or more of a first-control-mode instruction, a second-control-mode instruction, a third-control-mode instruction, a fourth-control-mode instruction and a fifth-control-mode instruction.

When the first-control-mode instruction is received, the end effector 20 is controlled according to a first control mode. In the first control mode, the pre-set unlocking condition may only include any one of a first unlocking condition, a second unlocking condition, a third unlocking condition, a fourth unlocking condition and a fifth unlocking condition in the above.

When the second-control-mode instruction is received, the end effector 20 is controlled according to a second control mode. In the second control mode, the pre-set unlocking condition may include any two of the first unlocking condition, the second unlocking condition, the third unlocking condition, the fourth unlocking condition and the fifth unlocking condition in the above.

When the third-control-mode instruction is received, the end effector 20 is controlled according to a third control mode. In the third control mode, the pre-set unlocking condition may include any three of the first unlocking condition, the second unlocking condition, the third unlocking condition, the fourth unlocking condition and the fifth unlocking condition in the above.

When the fourth-control-mode instruction is received, the end effector 20 is controlled according to a fourth control mode. In the fourth control mode, the pre-set unlocking condition may include any four of the first unlocking condition, the second unlocking condition, the third unlocking condition, the fourth unlocking condition and the fifth unlocking condition in the above.

When the fifth-control-mode instruction is received, the end effector 20 is controlled according to a fifth control mode. In the fifth control mode, the pre-set unlocking condition may include the first unlocking condition, the second unlocking condition, the third unlocking condition, the fourth unlocking condition and the fifth unlocking condition in the above.

Specifically, a manner of giving the control-mode instruction is not limited. For example, the control keys 60 further include a mode-switching key, and different control-mode instructions are given by switching and confirming of the mode-switching key.

In this way, operators can select a corresponding control-mode instruction according to their own usage habits, so that the control method can be adapted to use requirements of different operators and have strong adaptability.

To sum up, the embodiments of the present invention provide the intervention instrument 100, the control method of an intervention instrument, the computer-readable storage medium 80 and the controller 50. The control method of an intervention instrument includes: acquiring the position of the end effector 20 of the intervention instrument 100; and locking or unlocking the end effector 20 according to the relationship between the position where the end effector 20 is located and the pre-set position. For example, in the situation that the end effector 20 needs to be adjusted to the pre-set position, the end effector 20 may be manually controlled to be adjusted in the normal manner; and in the situation that the end effector 20 is adjusted to the pre-set position, the end effector 20 is locked. Therefore, the adjustment can be quickly, efficiently and accurately realized. For example, after sudden power-off and restart of the intervention instrument 100, or in a situation of accidental deadlock, it can be judged whether the position where the end effector 20 is located is the pre-set position. In a certain scenario, if the end effector 20 is located in the pre-set position, it indicates that work can be carried out normally, and the end effector 20 can be controlled to be unlocked. If the end effector 20 is not located in the pre-set position, it indicates that manual maintenance is required, and thus the end effector 20 can be controlled to remain locked. In a certain scenario, if the end effector 20 is located in the pre-set position, manual maintenance is required, and thus the end effector 20 can be controlled to remain locked. If the end effector 20 is not located in the pre-set position, it indicates that work can be carried out normally, and the end effector 20 can be controlled to be unlocked.

The above are merely for specific embodiments of the present invention, but the scope of protection of the present invention is not limited thereto. Changes or substitutions that could be easily conceived by any skilled person familiar with the technical field within the technical scope disclosed in the present invention should fall within the scope of protection of the present invention. Therefore, the scope of protection of the present invention should be based on the scope of protection of the claims.

### INDUSTRIAL APPLICABILITY

To sum up, the embodiments of the present invention provide the intervention instrument 100, the control method of an intervention instrument, the computer-readable storage medium 80 and the controller 50, which can realize quick, efficient and accurate adjustment of the end effector so as to realize locking or unlocking.

## Claims

1. A control method of an intervention instrument, **characterized by** comprising steps of:
acquiring a position of an end effector of the intervention instrument; and
locking or unlocking the end effector according to a relationship between the position where the end effector is located and a pre-set position.

2. The control method of an intervention instrument according to claim 1, **characterized in that** the pre-set position comprises a pre-set pulling-out position of the end effector; and
the step of locking or unlocking the end effector according to a relationship between the position where the end effector is located and a pre-set position comprises:
locking the end effector in a situation that the end effector moves to the pre-set pulling-out position, so that the end effector remains in the pre-set pulling-out position.

3. The control method of an intervention instrument according to claim 1, **characterized in that** the step of acquiring a position of an end effector of the intervention instrument comprises:
acquiring a swing angle of the end effector of the intervention instrument relative to a main body unit of the intervention instrument; and
determining, according to the swing angle, the position of the end effector of the intervention instrument.

4. The control method of an intervention instrument according to any one of claims 1-3, **characterized in that** the step of acquiring a position of an end effector of the intervention instrument comprises:
acquiring operating data of a drive source; and
determining the position of the end effector according to the operating data,
wherein the drive source is configured to drive the end effector of the intervention instrument to move relative to the main body unit.

5. The control method of an intervention instrument according to any one of claims 1-3, **characterized in that** the step of acquiring a position of an end effector of the intervention instrument comprises:
acquiring a position signal representing the end effector output by the position sensor; and
determining the position of the end effector according to the position signal.

6. The control method of an intervention instrument according to any one of claims 1-3, **characterized in that** the step of acquiring a position of an end effector of the intervention instrument comprises steps of:
acquiring operating data of a drive source; and determining a first position of the end effector according to the operating data, wherein the drive source is configured to drive the end effector of the intervention instrument to move relative to the main body unit;
acquiring the position signal representing the end effector output by the position sensor; and determining a second position of the end effector according to the position signal; and
determining, in a situation that the first position and the second position are consistent, the first position or the second position as the position of the end effector; and determining, in a situation that the first position and the second position are inconsistent, the position of the end effector according to a pre-set rule.

7. The control method of an intervention instrument according to claim 6, **characterized in that** the step of determining the position of the end effector according to a pre-set rule comprises:
determining an intermediate position of the first position and the second position as the position of the end effector.

8. The control method of an intervention instrument according to claim 7, **characterized in that** the step of locking the end effector comprises:
locking the drive source so as to lock the end effector,
wherein the drive source is configured to drive the end effector of the intervention instrument to move relative to the main body unit.

9. The control method of an intervention instrument according to any one of claims 1-8, **characterized in that** after the step of locking the end effector, the control method of an intervention instrument further comprises a step of:
unlocking the end effector in a situation that a pre-set unlocking condition is met.

10. The control method of an intervention instrument according to claim 9, **characterized in that** the step of unlocking the end effector in a situation that a pre-set unlocking condition is met comprises:
unlocking the end effector in a situation that a first trigger signal is acquired,
wherein the first trigger signal is triggered by a control key.

11. The control method of an intervention instrument according to claim 9 or 10, **characterized in that** the step of unlocking the end effector in a situation that a pre-set unlocking condition is met comprises:
unlocking the end effector in a situation that a second trigger signal is acquired,
wherein the second trigger signal represents that under control of a control key, the end effector moves away from a pre-set pulling-out position.

12. The control method of an intervention instrument according to any one of claims 9-11, **characterized in that** the step of unlocking the end effector in a situation that a pre-set unlocking condition is met comprises:
unlocking the end effector in a situation that a first trigger-releasing signal is acquired and a third trigger signal is acquired,
wherein the first trigger-releasing signal represents that an action of one of the control keys 60 being triggered to execute controlling the end effector to move towards a pre-set pulling-out position is released, and the third trigger signal represents that another control key is triggered.

13. The control method of an intervention instrument according to any one of claims 9-12, **characterized in that** the step of unlocking the end effector in a situation that a pre-set unlocking condition is met comprises:
unlocking the end effector in a situation that a second trigger-releasing signal is acquired and a fourth trigger signal is acquired after first pre-set duration,
wherein the second trigger-releasing signal represents that a control key is triggered by a set action to control the end effector to rotate in a single direction so as to be unlocked; and the fourth trigger signal represents that the control key is triggered again by the set action to control the end effector to rotate in a single direction.

14. The control method of an intervention instrument according to any one of claims 9-13, **characterized in that** the step of unlocking the end effector in a situation that a pre-set unlocking condition is met comprises:
unlocking the end effector in a situation that duration of a trigger signal of a control key acquired is longer than or equal to second pre-set duration.

15. An intervention instrument, **characterized by** comprising:
a main body unit;
an end effector, wherein the end effector is movable relative to the main body unit;
a drive source, wherein the drive source is configured to drive the end effector of the intervention instrument to move relative to the main body unit; and
a controller, wherein the controller is in communication connection with the drive source, and the controller is configured to implement the control method of an intervention instrument according to any one of claims 1-14.

16. The intervention instrument according to claim 15, **characterized in that** the controller is further configured to determine the position of the end effector according to operating data of the drive source; and configured to lock or unlock the end effector according to the relationship between the position where the end effector is located and the pre-set position.

17. The intervention instrument according to claim 15 or 16, **characterized in that** the intervention instrument further comprises a position sensor, wherein the position sensor is mounted to the main body unit or the end effector; the position sensor is in communication connection with the controller, and the position sensor is configured to detect a position of the end effector relative to the main body unit; and
the controller is configured to lock or unlock the end effector according to the relationship between the position where the end effector is located and the pre-set position.

18. The intervention instrument according to any one of claims 15-17, **characterized in that** the intervention instrument further comprises a control key in communication connection with the controller; and
the controller is configured to control the drive source according to a signal of the control key.

19. The intervention instrument according to claim 18, **characterized in that** a plurality of control keys are provided; and
at least one of the control keys is distributed on at least one side of the main body unit; or
a plurality of the control keys are distributed on the same side of the main body unit, and among a plurality of the control keys located on the same side, at least one of the control keys is configured to control the end effector to rotate forward, and at least one of the control keys is configured to control the end effector to rotate reversely.

20. The intervention instrument according to any one of claims 15-19, **characterized in that** the intervention instrument further comprises an intermediate transmission unit, and the drive source drives the end effector to move relative to the main body unit through the intermediate transmission unit.

21. The intervention instrument according to claim 20, **characterized in that** the intermediate transmission unit comprises a lead-screw mechanism, a traction element, a gear-rack mechanism and a gear assembly, wherein
the lead-screw mechanism comprises a screw, a nut and a slider mounted to the main body unit, the drive source is connected to the screw, the screw is in threaded connection with the nut, and the nut is connected to the slider;
the gear-rack mechanism comprises a limiting rack and a first gear mounted to the main body unit, wherein the limiting rack is connected to the slider through the traction element, and the limiting rack meshes with the first gear; and
the gear assembly comprises a second gear and a joint gear, wherein the second gear is mounted to the main body unit, the second gear meshes with the first gear, the joint gear is mounted to the end effector, and the joint gear meshes with the second gear.

22. A computer-readable storage medium, **characterized by** storing a computer program/instruction, wherein the computer program/instruction, when executed, implements the control method of an intervention instrument according to any one of claims 1-14.

23. A controller, **characterized by** comprising a computer program/instruction, wherein the computer program/instruction, when executed, implements the control method of an intervention instrument according to any one of claims 1-14.
